# EUROPEAN PATENT SPECIFICATION

(11) **EP 1 392 348 B1**
(45) Date of publication and mention of the grant of the patent: **09.07.2008**
(21) Application number: 02735312.7
(22) Date of filing: 27.04.2002
(51) Int. Cl.: A61K 38/09, A61P 25/28

(54) **TREATMENT OF DEMENTIA AND NEURODEGENERATIVE DISEASES WITH INTERMEDIATE DOSES OF LHRH ANTAGONISTS**
NIEDRIGDOSIERTE LHRH ANTAGONISTEN ZUR BEHANDLUNG VON NEURODEGENERATIVEN KRANKHEITEN, INSBESONDERE ALZHEIMER KRANKHEIT
TRAITEMENT DE LA DEMENCE ET DE MALADIES NEURODEGENERATIVES PAR DES DOSES INTERMEDIAIRES D'ANTAGONISTES DE LHRH

(30) Priority: 30.04.2001 US 287434 P
(43) Date of publication of application: 03.03.2004
(73) Proprietor: AEterna Zentaris GmbH, 60314 Frankfurt (DE)
(72) Inventor: ENGEL, Jürgen, 63755 Alzenau (DE); VOEGELI, Rainer, 82407 Wielenbach (DE)
(86) International application number: PCT/EP2002/004677
(87) International publication number: WO 2002/102401

(56) References cited:
- WO-A-00/55190
- WO-A-00/69859
- WO-A-01/29044
- WO-A-02/02533
- WO-A-97/44339
- CA-A- 2 309 395

## Description

The present invention relates to the treatment of dementia and neurodegenerative diseases with monthly single doses of LHRH antagonists which do not cause a castration.

FURUYA, Shuichi et al. in WO 01/78780 teach preventives and remedies for Alzheimer's disease containing a compound having GnRH antagonism have effects of preventing and treating Alzheimer's disease with little toxicity.

It has been shown in a study by Bowen R.L et al. that serum concentrations of follicle-stimulating hormone (FSH) and luteinizing hormone (LH) were significantly higher in individuals suffering of dementia, e.g. Alzheimer's disease. Bowen R.L. et al. propose in their patent application CA 2,309,395 (US priority June 4, 1999, 09/326, 180) to lower FSH and LH to minimal levels by the use of castrating doses of analogues of the LH-releasing hormone (LHRH), either super-agonists or antagonists.
This treatment would be accompanied by highly undesirable side effects as lowering sex hormone levels to castration levels would result in loss or reduction of libido, sexual desire and sexual potency. In men and pre-menopausal women this treatment would also result in the typical symptoms of drop of sex hormones like hot flushes, etc. Women would additionally suffer from loss of bone minerals that would limit the treatment.
These side effects could be reduced by hormone replacement therapy.

It has been found now that the treatment with monthly single doses of LHRH antagonists results in a sub-maximal lowering of FSH and LH to normal levels that leaves sex hormone levels above the castration treshold.
This treatment is highly advantageous as it gives the desired results of normalising FSH and LH levels without the undesirable side-effects of sex hormone blockade. Thus the additional treatment of sex hormone replacement becomes superfluous.

The present invention relates to the treatment of dementia and neurodegenerative diseases with monthly single doses of LHRH antagonists, wherein the antagonist is preferably cetrorelix, teverelix, antide or abarelix. The antagonist can also be the LHRH antagonist D-63 153 (Ac-D-Nal-D-pCl-Phe-D-Pal-Ser-N-Me-Tyr-D-Hci-Nle-Arg-Pro-D-Ala-NH2) as described in the PCT application WO 00/55190 Al.

The mentioned LHRH antagonists can also exhibit a heterocyclic skeletal structure. Such peptidomimetics can be for example
- 1-[7-Chloro-3-(3,5-dimethyl-phenyl)-2-oxo-4-(2-piperidin-2-yl-ethoxy)-1,2-dihydro-quinolin-6-yl]-3-pyridin-2-yl-urea (described in WO 97/44339),
- 3-[Benzyl-methyl-amino)-methyl]-2-tert-butyl-8-(2-fluoro-benzyl)-6-(3-methoxyphenyl)-7-methyl-8H-imidazo[1,2-a]pyrimidin-5-one (described in WO 01/29044),
- 2-(2,5-Dimethyl-furan-3-yl)-8-(2-fluoro-benzyl)-3-([methyl-(2-pyridin-2-yl-ethyl)-amino]-methyl)-5-oxo-5,8-dihydro-imidazo[1,2-a]pyrimidine-6-carboxylic acid 1-ethyl-propylester (described in WO 00/69859),
- 3-((2-[2-(3,5-Difluoro-phenyl)-1-(2-methoxy-benzoyl)-2-oxo-ethylidene]-2,3-dihydro-1H-benzoimidazol-5-yl-amino)-methyl)-benzonitrile (described in WO 02/02533).

The LHRH antagonist is given in a monthly single dose of 60 to 100 mg and the treatment is repeated monthly, two-monthly or lasting several months.

In a preferred embodiment the LHRH antagonist is given in a monthly single dose of 60 mg per month and the treatment is repeated monthly, two-monthly or lasting several months.

Pharmaceutical formulations of the LHRH antagonist suitable for the therapeutic management of dementia and neurodegenerative diseases may be for example
a) acetate salt formulations of the active compounds in the concentration of 1 mg/1 ml or lower where the lyophilisate powder may be dissolved in water for injection or in gluconic acid;
b) acetate salt formulations of the active compounds in the concentration of 1.5 mg/1 ml to 5.0 mg/1 ml, preferably 2.5 mg/1 ml where the lyophilisate powder may be dissolved in water for injection or in gluconic acid;
c) pamoate salt formulations of the active compounds in the concentration of 10 mg/1 ml to 30 mg/1 ml, preferably 15 mg/1 ml where the lyophilisate powder may be dissolved in gluconic acid or in water for injection.

Suitable excipients and dosage forms are for example described by K.H. Bauer, K.-H. Frömming and C. Führer, Lehrbuch der Pharmazeutischen Technologie, 6th edition, Stuttgart 1999, pages 163-186 (excipients) and pages 227-386 (dosage forms), including the references as cited therein.

The LHRH antagonist can be administered for example subcutaneous, oral, intramuscular or inhalative.

The disease as mentioned, for example can be treated in accordance with the following scheme.

### Example

In one embodiment of the invention a single dose of 60 mg of cetrorelix is administered by injection per month. The treatment is continued monthly.
In another embodiment the treatment is continued two-monthly or lasting several months alter the administration of the single dose.

## Claims

1. Use of a LHRH antagonist for the preparation of a medicament for the treatment of dementia and neurodegenerative diseases in humans, **characterized in that** the LHRH antagonist is given in a monthly single dose in the range of 60 - 100 mg per month **and in that the LHRH antagonist is cetrorelix, teverelix, antide, abarelix, D-63153 (Ac-D-Nal-D-pCl-Phe-D-Pal-Ser-N-Me-Tyr-D-Hci-Nle-Arg-Pro-D-Ala-NH₂) or a peptidomimetic.**

2. Use according to claim 1, **characterized in that** the monthly single dose is 100 mg LHRH antagonist.

3. Use according to claim 1, **characterized in that** the monthly single dose is about 60 mg LHRH antagonist.

4. Use according to one of claims 1 to 3, **characterized in that** the treatment is repeated monthly, two-monthly or lasting several months.

5. Use according to one of claims 1 to 4, **characterized in that** the treated disease is Alzheimer's disease.

6. 7. Use according to any of claims 1 to 5 in which the peptidomimetic is a compound
- 1-[7-Chloro-3-(3,5-dimethy)-phenyl)-2-oxo-4-(2-piperidin-2-yl-ethoxy)-1.2-dihydro-quinolin-6-yl]-3-pyridin-2-yl-urea
- 3-[Benzyl-methyl-amino)-methyl]-2-tert-butyl-8-(2-fluoro-benzyl)-6-(3-methoxy-phenyl)-7-methyl-8H-imidazo[1,2-a]pyrimidin-5-one
- 2-(2,5-Dimethyl-furan-3-yl)-8-(2-fluoro-benzyl)-3-([methyl-(2-pyridin-2-yl-ethyl)-amino]-methyl)-5-oxo-5,8-dihydro-imidazo[1,2-a]pyrimidine-6-carboxylic acid 1-ethyl-propylester or
- 3-((2-[2-(3,5-Difluoro-phenyl)-1-(2-methoxy-benzoyl)-2-oxo-ethylidene]-2,3-dihydro-1H-benzoimidazol-5-yl-amino)-methyl)-benzonitrile.

## Patentansprüche

1. Verwendung eines LHRH-Antagonisten bei der Herstellung eines Medikaments zur Behandlung von Demenz und neurodegenerativen Erkrankungen bei Menschen, **dadurch gekennzeichnet, dass** der LHRH-Antagonist in einer monatlichen Einzeldosis im Bereich von 60 bis 100 mg pro Monat gegeben wird und dass der LHRH-Antagonist Cetrorelix, Teverelix, Antid, Abarelix, D-63153 (Ac-D-Nal-D-pCl-Phe-D-Pal-Ser-N-Me-Tyr-D-Hci-Nle-Arg-Pro-D-Ala-NH₂) oder ein Peptidomimetikum ist.

2. Verwendung gemäß Anspruch 1, **dadurch gekennzeichnet, dass** es sich bei der monatlichen Einzeldosis um 100 mg LHRH-Antagonist handelt.

3. Verwendung gemäß Anspruch 1, **dadurch gekennzeichnet, dass** es sich bei der monatlichen Einzeldosis um etwa 60 mg LHRH-Antagonist handelt.

4. Verwendung gemäß einem der Ansprüche 1 bis 3, **dadurch gekennzeichnet, dass** die Behandlung monatlich, zweimonatlich oder über mehrere Monate wiederholt wird.

5. Verwendung gemäß einem der Ansprüche 1 bis 4, **dadurch gekennzeichnet, dass** die behandelte Erkrankung die Alzheimersche Erkrankung ist.

6. Verwendung gemäß einem der Ansprüche 1 bis 5, wobei es sich bei dem Peptidomimetikum um eine Verbindung
- 1-[7-Chlor-3-(3,5-dimethylphenyl)-2-oxo-4-(2-piperidin-2-ylethoxy)-1,2-dihydrochinolin-6-yl]-3-pyridin-2-ylharnstoff,
- 3-[Benzylmethylamino)methyl]-2-*tert*-butyl-8-(2-fluorbenzyl)-6-(3-methoxyphenyl)-7-methyl-8H-imidazo[1,2-a]pyrimidin-5-on,
- 2-(2,5-Dimethylfuran-3-yl)-8-(2-fluorbenzyl)-3-([methyl-(2-pyridin-2-ylethyl)amino]methyl)-5-oxo-5,8-dihydroimidazo[1,2-a]pyrimidin-6-carbonsäure-1-ethylpropylester oder
- 3-((2-[2-(3,5-Difluorphenyl)-1-(2-methoxybenzoyl)-2-oxoethyliden]-2,3-dihydro-1H-benzoimidazol-5-ylamino)methyl)benzonitril
handelt.

## Revendications

1. Utilisation d'un antagoniste de la LHRH pour la préparation d'un médicament pour le traitement de la démence et des maladies neurodé-génératives chez l'humain,
**caractérisée en ce que**
l'antagoniste de la LHRH est administré à raison d'une dose unique mensuelle dans la plage de 60-100 mg par mois, et **en ce que** l'antagonisme de la LHRH est choisi parmi les cétrorelix, teverelix, antide, abarelix, D-63153 (Ac-D-Nal-D-pCI-Phe-D-Pal-Ser-N-Me-Tyr-D-Hci-Nle-Arg-Pro-D-Ala-NH₂, ou est un peptidomimétique.

2. Utilisation selon la revendication 1,
**caractérisée en ce que**
la dose unique mensuelle est de 100 mg d'antagoniste de la LHRH.

3. Utilisation selon la revendication 1,
**caractérisée en ce que**
la dose unique mensuelle est d'environ 60 mg de l'angoniste de la LHRH.

4. Utilisation selon l'une des revendications 1 à 3,
**caractérisée en ce que**
le traitement est répété tous les mois, tous les deux mois, ou dure plusieurs mois.

5. Utilisation selon l'une des revendications 1 à 4,
**caractérisée en ce que**
la maladie traitée est la maladie d'Alzheimer.

6. Utilisation selon l'une quelconque des revendications 1 à 5 selon laquelle le peptidomimétique est :
- la 1-[7-chloro-3-(3,5-diméthyl-phényl)-2-oxo-4-(2-piperidine-2-yl-éthoxy)-1, 2-dihydroquinoline-6-yl]-3-pyridine-2-yl-urée,
- la 3-[benzyl-méthyl-amino)-méthyl]-2-tert-butyl-8-(2-fluoro-benzyl)-6-(3-méthoxy-phényl)-7-méthyl-8H-imidazol[1,2-a]pyrimidine-5-one,
- le 1-éthyl-propylester d'acide (2-(2,5-diméthyl-furane-3-yl)-8-(2-fluoro-benzyl)-3-([méthyl-(2-pyridine-2-yl-éthyl)-amino]-méthyl)-5-oxo-5,8-dihydro-imidazo[1,2-a]pyrimidine-6-carboxylique, ou
- le 3-((2-[2(3,5-difluoro-phényl)-1-(2-méthoxy-benzoyl)-2-oxo-éthylidène]-2,3-dihydro-1H-benzoimidazol)-5-yl-amino)-méthyl)-benzonitrile.
